# EUROPEAN PATENT SPECIFICATION

(11) **EP 1 949 060 B1**
(45) Date of publication and mention of the grant of the patent: **02.01.2019**
(21) Application number: 06816973.9
(22) Date of filing: 12.10.2006
(51) Int. Cl.: G01L 15/00, G01N 11/08, G01N 30/02, G01N 30/32, G01N 33/44, G01N 30/62

(54) **IMPROVED MULTI-CAPILLARY VISCOMETER APPARATUS AND METHOD**
VERBESSERTE MEHRFACHKAPILLAR-VISKOMETERVORRICHTUNG UND VERFAHREN
VISCOSIMETRE MULTICAPILLAIRE AMELIORE ET PROCEDE

(30) Priority: 12.10.2005 US 249839
(43) Date of publication of application: 30.07.2008
(73) Proprietor: Malvern Panalytical Inc., Westborough, MA 01581 (US)
(72) Inventor: TITTERTON, Alan, Yarm TS15 0HL (GB)
(74) Representative: Barker Brettell LLP
(86) International application number: PCT/US2006/040342
(87) International publication number: WO 2007/044954

(56) References cited:
- WO-A1-96/34269
- US-A- 4 578 990
- US-A- 4 627 271
- US-A- 5 637 790
- US-B2- 6 708 553
- US-B2- 6 712 085
- US-B2- 6 877 361
- G. MASON ET AL: "Capillary viscometry by perturbation of flow and composition", CHEMICAL ENGINEERING SCIENCE, vol. 53, no. 15, 1 August 1998 (1998-08-01), pages 2665-2674, XP055084995, ISSN: 0009-2509, DOI: 10.1016/S0009-2509(98)00106-7
- P. A. RUSSELL ET AL: "Perturbation viscometer to measure the viscosity gradients of gas mixtures", AICHE JOURNAL, vol. 49, no. 8, 1 August 2003 (2003-08-01) , pages 1986-1994, XP055084963, ISSN: 0001-1541, DOI: 10.1002/aic.690490809

## Description

### FIELD OF THE INVENTION

The present invention relates to multi-capillary viscometers used to measure and analyze the viscosity of a sample solution. The viscosity analysis circuit may be used in conjunction with Gel Permeation Chromatography ("GPC") or Size Exclusion Chromatography ("SEC") to determine other properties of the sample such as molecular size or weight distributions.

Mason et al, in "Capillary viscometry by perturbation of flow and composition", Chemical Engineering Science, Vol. 53, No. 15, pp 2665-2674, 1998, disclose a technique for making viscosity measurements on gas mixtures in which the composition and flowrate of a mixture flowing through a capillary tube are perturbed by adding a small stream of perturbation gas and a change in viscosity measured from a ratio between two resulting pressure changes.

### BRIEF DESCRIPTION OF DRAWINGS

FIG. 1 illustrates a schematic view of a general multi-capillary viscometer.
FIG. 2a illustrates a block diagram of one embodiment of the apparatus with two delay volume components and a fluid diverting component in orientation A.
FIG. 2b illustrates a block diagram of one embodiment of the apparatus with two delay volume components and a fluid diverting component in orientation B.
FIG. 3a is a block diagram of one embodiment of the subcircuit of the apparatus with two delay volume components and a fluid diverting component in orientation A.
FIG. 3b is a block diagram of one embodiment of the subcircuit of the apparatus with two delay volume components and a fluid diverting component in orientation B.
FIG. 4a is a block diagram of one embodiment of the subcircuit of the apparatus with a single delay volume component and a fluid diverting component in orientation A.
FIG. 4b is a block diagram of one embodiment of the subcircuit of the apparatus with a single delay volume component and a fluid diverting component in orientation B.

### DETAILED DESCRIPTION OF THE EMBODIMENTS

For a further understanding of the nature, function, and objects of the present invention, reference is made to the following detailed description taken in conjunction with the accompanying drawings. Detailed descriptions of embodiments of the apparatus are provided herein, as well as modes of carrying out and employing the embodiments of the present invention. It is to be understood, however, that the present apparatus may be embodied in various forms. The description provided herein relates to the common components of sample capillary, delay volume components, reference capillary and diverter valve which may form only part of a more complex circuit. Therefore, specific details disclosed herein are not to be interpreted as limiting, but rather as a basis for the claims and as a representative basis for teaching one skilled in the art to employ the present embodiments of one form of the apparatus or method in virtually any appropriately detailed system, structure or manner. The embodiments described herein are considered illustrative of both the processes taught by the described embodiments of products and articles of manufacture yielded in accordance with the present embodiments of one form of the apparatus. The inventive device and method can be used in a multi-capillary viscometer for which the basic operating principles are disclosed in U.S. Patent No. 4,463,598 to Haney, in U.S. Pat. Nos. 4,627,271 and 4,578,990 to Abbott, et al, and in U.S. Patent No. 5,637,790 to de Corral.

In the prior art, multi-capillary viscometers are constructed and operated such that a reference flow of solvent may be maintained during a measurement by inserting a delay volume component in front of a reference capillary. The delay volume component initially contains pure solvent at the beginning of the analysis. It provides a continuing flow of solvent to a reference capillary while sample flows though a sample capillary. A limitation of such prior art multi-capillary viscometers, whether used only for viscosity measurements or in combination with other detectors in GPC or SEC analysis, is what is typically referred to as a breakthrough peak. The breakthrough peak is an unwanted instrument response resulting from the discharge of sample fluid from the delay volume component through a reference capillary after pressures have been detected or sensed that are useful in determining information about the viscosity of the sample. The breakthrough peak can last for a substantial period of time after the measurement process is completed because the delay volume spreads the peak, thereby delaying the measurement of the next sample.

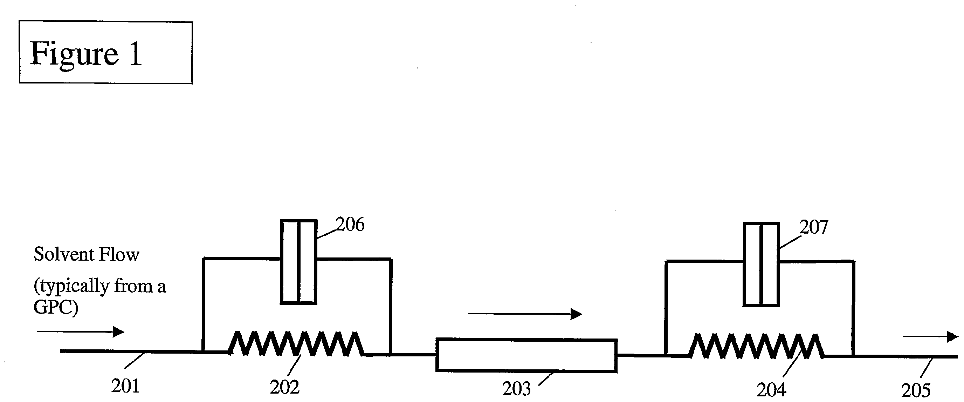

The cause of the breakthrough peak effect can be appreciated by reference to Figure 1 accompanied with the following description. Figure 1 illustrates schematically a simple multi-capillary viscometer and can be described as an operating circuit. The operating circuit consists of an inlet tube 201 coupled in series to a narrower bore capillary tube 202 referred to as the sample capillary, a delay volume component 203 which has a hold-up volume of significantly large capacity, a second narrow bore capillary tube 204 referred to as the reference capillary, and finally an exit tube 205. When a fluid such as a solvent is pumped through the circuit, a differential pressure will be generated, mainly across the capillary tubes since they have a narrower cross-sectional area than the connecting tubing and other components in the circuit. The pressure across each capillary may be monitored continuously by means of suitable transducers 206, 207. A sample solution is injected into the flowing solvent stream and is carried through the circuit. Its passage through the sample capillary 202 will typically cause an increased pressure drop because of the viscosity of the sample and the increased pressure drop will be detected by the transducer 206 across the respective capillary 202. However, the pressure monitored by the transducer 207 across the reference capillary 204 will remain substantially unaltered since it will still be receiving solvent eluting from the delay volume component 203. The relative viscosity may be mathematically derived using the ratio of the pressure drop across the sample capillary 202 to the pressure drop across the reference capillary 204 as described in, for example, U.S. Pat. Nos. 4,627,271 and 4,578,990, Abbott, et al. The relative viscosity will increase as sample flows into the viscometer.

The volume of the delay volume component is selected to be sufficient to supply the reference capillary 204 with solvent during the measurement process. Eventually, all the sample solution will emerge from the sample capillary 202 and enter the delay volume component 203. The pressure across the sample capillary 202 will return to the baseline value and the useful part of the measurement cycle is over. However, the sample will eventually progress all the way through the delay volume component 203 and enter the reference capillary 204 where it will cause a rise in pressure, measured by the transducer 207. This increase in pressure in turn causes a decrease in the measured relative viscosity, which is the cause of the breakthrough peak.

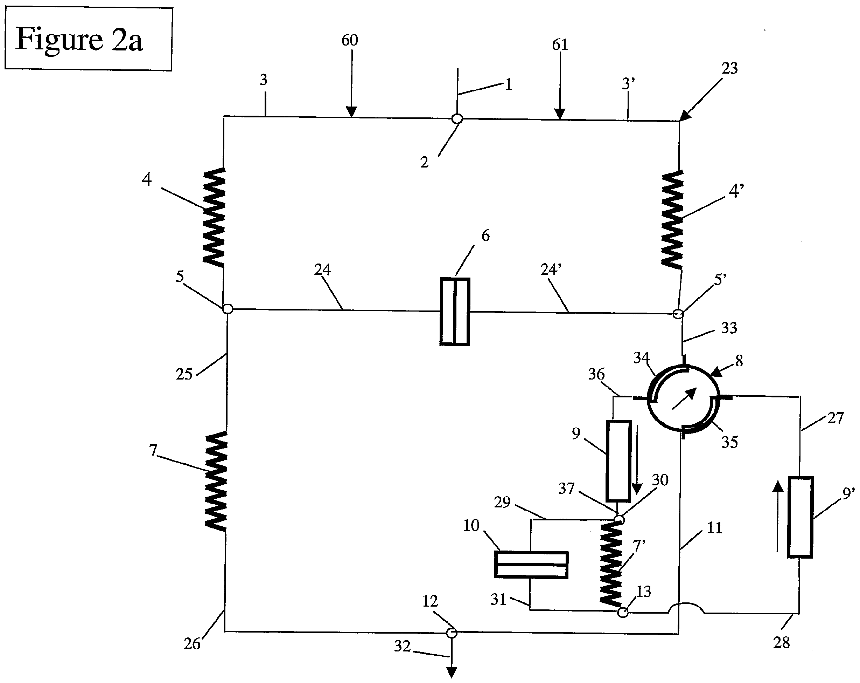

Figure 2a illustrates one embodiment of the present embodiments of one form of the apparatus comprising a fluid viscosity measurement circuit 23 having components connected together preferably using conventional tubing connectors, fittings or unions and connecting tubing. The fluid viscosity measurement circuit 23 preferably has a fluid insertion or injection tube 1 through which a fluid is inserted. Fluid viscosity measurement circuit 23 preferably has a first fluid flow circuit 60 and a second fluid flow circuit 61.

In the embodiment illustrated by Figure 2a, the fluid insertion or injection tube 1 attaches to a split junction 2, which can include but is not limited to a T-bridge split junction. The split junction 2 is attached to two lengths of pipe or tubing 3, 3'. The two lengths of pipe or tubing 3, 3' are attached to the split junction 2 so as to allow a fluid to move through the split junction 2 into the length of pipe or tubing 3 and the other length of pipe or tubing 3'. The length of pipe or tubing 3 is connected between the split junction 2 and a first capillary 4 in the flow circuit 60. The first capillary 4 is preferably a conventional fluid capillary, which is preferably a tube, having a relatively small inside diameter (typically, but not limited to, the range of about 0.023 mm (0.009") to 0.36 mm (0.014")) in comparison to the relatively large inside diameter of other fluid components in the circuit (typically, but not limited to 1 mm (0.04") or more for connecting tubing and fittings and 1.57 mm (0.062") or more for delay volumes) or more for delay volumes). The first capillary 4 serves to provide a flow restriction within the fluid viscosity measurement circuit. The terms "capillaries" or "capillary" are used throughout this application in their normal and customary manner to include, for example, any structure with a cross-sectional hollow portion having a relatively small inside diameter to produce a pressure drop higher than that produced by other individual components of the fluid viscosity measurement circuit.

The length of pipe or tubing 3' is connected between the split junction 2 and a first capillary 4' of the second flow circuit 61. The capillaries 4, 4' are preferably conventional fluid capillaries. The first capillary 4 is preferably connected to a second split junction 5. The first capillary 4' is preferably connected to a second split junction 5'. The first split junction 5 and the second split junction 5' are preferably connected to a transducer tube or line 24, 24', respectively. The transducer tubes or lines 24, 24' are preferably connected to the second split junctions 5, 5', respectively, and to a transducer 6. The terms "transducer" or "transducers" are used throughout this application in their normal and customary manner to include, for example, any structure or apparatus operable for sensing or measuring fluid differential pressures and, more particularly, differential pressure transducers of the type described in the Abbot, *et al.* and De Coral patents wherein two cavities are separated by a diaphragm which is deflected by a pressure difference in the cavities to produce an electrical signal proportional to the pressure differential.

The transducer 6, and all transducers referred to herein, is preferably connected in a "dead-end" manner, such that only the inlet ports of the transducers remain open after the transducer lines and cavities are filled and purged for operation, and pressure is transmitted by static fluid in the transducer lines and cavities to the transducer diaphragm. The transducer 6 and all transducers referred to herein may also be connected in a "flow-through" manner, wherein inlet, outlet or purge ports of each cavity of the transducer are connected such that fluid flowing through one or more of the other components in the circuit also flows through the transducer cavities, and fluid pressure is transmitted to the transducer diaphragm by the fluid flowing through the transducer cavity.

The split junction 5 preferably connects to a fluid tube 25. The fluid tube 25 preferably connects to a capillary 7. The capillary 7 preferably connects to another fluid tube 26. The fluid tube 26 preferably connects to a split junction 12. The split junction 12 preferably connects to yet another fluid tube 32.

The split junction 5' in the second fluid flow circuit 61 preferably connects to a fluid tube 33. The fluid tube 33 preferably connects to a fluid path diverter valve 8. The fluid path diverter valve 8 preferably contains a plurality of fluid pathways 34, 35. The terms "diverter valve" or "valve" are used in this application in their normal and customary manner, and by way of example, refers to any valve or structure operable to selectively direct or align the flow of fluid from one fluid pathway to another and may include, but is not limited to, a valve operable for that purpose in a prescribed or automated fashion, which may be, but is not limited to, electrical, pneumatic or timed operation or activation. The term "diverter valve" as used in this application can be, but is not limited to a 4-port, 2 position plug valve, such as Hamilton HV-86779. As shown in Figure 2a, a first fluid pathway 34 is connected to a first fluid tube 36, and a second fluid pathway 35 is connected to a second fluid tube 27. The first fluid tube 36 is connected to a first delay volume 9. The first delay volume 9 is connected to a downstream fluid tube 37. The term "delay volume" is used in this application in its normal and customary manner, and for example, includes any means of delaying a fluid's arrival at another point in the fluid circuit, which may include, but is not limited to, increased volume tubing or reservoirs. A typical delay volume can include, but is not limited to a packed column. The downstream fluid tube 37 is preferably connected to a split junction 30. A capillary 7' is also connected to the split junction 30. The capillary 7' can be, but is not necessarily, substantially identical to the corresponding capillary 7. A transducer line or tube 29 is also attached to the split junction 30.

The transducer line or tube 29 is preferably connected to a transducer 10. The transducer 10 is preferably a conventional transducer utilized in measuring fluid viscosity. The transducer 10 is preferably connected in the "dead-end" manner described above for the center transducer 6. The transducer 10 and the center transducer 6 are preferably substantially identical. The transducer 10 is also connected to a transducer line or tube 31 such that the connections of transducer line or tube 29 and transducer line or tube 31 are on substantially opposite sides of the diaphragm associated with the transducer 10. The transducer line or tube 31 is connected to a split junction 13. The capillary 7' is likewise connected to the split junction 13. A fluid tube line 28 is preferably connected to the split junction 13 and to a second delay volume 9'. The first delay volume 9 and the second delay volume 9' are preferably substantially identical. The second delay volume 9' is preferably connected on its opposite end to a fluid tube 27. The fluid tube 27 is preferably connected to the second fluid pathway 35 of the fluid path diverter valve 8. The second fluid pathway 35 is also preferably connected on its opposite end to a fluid tube 11. The fluid tube 11 is connected to the split junction 12.

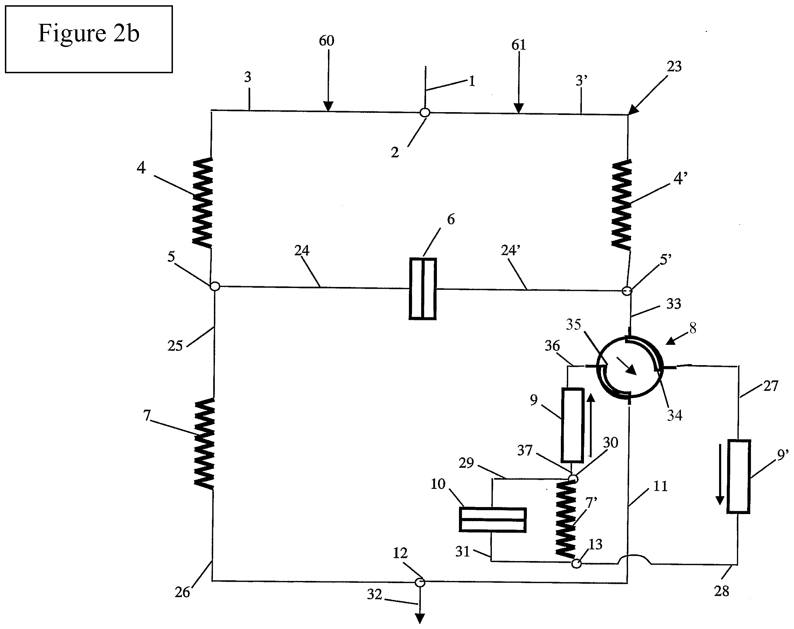

Figure 2b, illustrates another embodiment of an apparatus of the present invention similar to the embodiment illustrated in Figure 2a. The embodiment illustrated in Figure 2b is similar to the embodiment illustrated in Figure 2a except in the embodiment in Figure 2b, the fluid path diverter valve 8 is rotated to another position. Particularly, the fluid path diverter valve 8 is positioned such that the fluid pathway 34 is aligned between the respective fluid tubes 33, 27, and the fluid pathway 35 is aligned between the respective fluid tubes 11, 36.

As appreciated by one skilled in the art, the embodiment of the apparatus disclosed in Figure 2a operates in, but is not limited to, substantially the following manner. When in pre-sample testing mode, a stream of reference fluid or solvent runs through the system and follows one of a plurality of pathways. As the solvent flows into the system through the injection tube 1, the solvent moves until it approaches the split junction 2, at which point the fluid stream of solvent is divided to flow into both fluid tubes 3, 3'. The solvent then flows through the two first capillaries 4 and 4' and approaches the two split junctions 5, 5', respectively. The fluid pressure at the two split junctions 5, 5', respectively, is transmitted by the fluid in the transducer tubes or lines 24, 24' to opposite sides of the transducer 6. The fluid that flows into the fluid tube 25, adjacent to the second split junction 5, proceeds to flow through the capillary 7, through the fluid tube 26 and then to the split junction 12. Alternately, the fluid that flows from the fluid tube 33, adjacent to the second split junction 5', flows into the first fluid pathway 34. The fluid then flows into the fluid tube 36 and then into the delay volume 9. The fluid stream then flows into the fluid tube 37, which connects to the split junction 30, and then into the capillary 7'. The fluid pressure at the split junction 30 is transmitted by the fluid in the transducer line or tube 29 to one side of the transducer 10. The fluid entering the split junction 30 from the delay volume 9 flows through the capillary 7' to the split junction 13. The pressure of the fluid at the split junction 13 is transmitted by the fluid in transducer line or tube 31 to one side of the transducer 10, thus enabling the transducer 10 to produce a signal substantially corresponding to the differential pressure across the capillary 7' (the difference in fluid pressures upstream and downstream of the capillary 7'). The fluid flow, when it reaches the split junction 13, flows into the fluid tube 28, then into the delay volume 9' and then into the fluid tube 27. The fluid flows into the fluid pathway 35 of the fluid path diverter valve 8 and then into the fluid tube 11. The fluid then flows into the split junction 12. The fluids in the fluid tube 26 and the fluid tube 11 meet at the split junction 12, then exit via the fluid tube 32 which is connected to the split junction 12. Reference fluid may be flowed continuously through the measurement circuit to allow base line readings to be developed from the transducers 6, 10 with the measurement circuit full of flowing reference fluid.

With the fluid diverter valve 8 positioned as disclosed in Figure 2b, the measurement circuit operates in a similar manner as described in Figure 2a. The fluid flows from the tube 33, into the fluid pathway 34 and then into the fluid tube 27. The fluid then enters the delay volume 9' and flows into the fluid tube 28. The fluid then flows into the split junction 13 connecting the transducer line or tube 31 and the capillary 7'. The pressure of the fluid at the split junction 13 is transmitted by the fluid in the transducer line or tube 31 to one side of the transducer 10. The fluid that flows through the capillary 7' enters split junction 30 and then into the fluid tube 37. The pressure of the fluid at the split junction 30 is transmitted by the fluid in the transducer line or tube 29 to one side of the transducer 10. The fluid exiting the fluid tube 37 flows into the delay volume 9. The fluid then flows from the delay volume 9 into the fluid tube 36, which is connected to the fluid pathway 35. The fluid flows into the fluid pathway 35, which is connected to the fluid tube 11 and then to the split junction 12.

To measure the viscosity of a sample fluid with the viscosity measurement circuit of the present invention as illustrated in Figure 2a, the sample is injected or added into the reference fluid at or upstream of the inlet to the measurement circuit at the injection tube 1. The sample then flows in solution with the reference fluid along the same fluid pathway as described above for Figure 2a. The delay volume 9 delays the arrival of the sample solution at the capillary 7' such that the differential pressures across the corresponding capillaries 7, 7' may be sensed by the transducers 6, 10 when the capillary 7 contains sample material and the capillary 7' contains only the solvent or reference fluid. Hence, the pressure drop sensed by the sample transducer 6 will be different than the pressure drop sensed at that time by the reference transducer 10 because the reference transducer 10 will still be sensing a differential pressure associated with the pure solvent in the capillary 7'. This enables the transducers 6, 10 to produce signals substantially corresponding to the pressure drops across the capillary containing pure solvent and the capillary containing the sample solution for use in determining relative viscosity, which may be mathematically related to other characterizations of the sample's properties, such as intrinsic viscosity, inherent viscosity, specific viscosity and reduced viscosity, by methods known in the art, such as is described in, for example, the Abbott, *et al.* patent and the De Corral patent. The transducer signals may also be used in combination with the signals produced by a refractometer or similar concentration detector, which is part of a GPC system, to determine other information about the sample's properties such as, for example, molecular weight distribution.

After the relative differential pressure information is obtained, the fluid path diverter valve 8 may be switched to realign the valve, in a conventional fashion, such that the flow of fluid through the reference capillary 7' is reversed as illustrated in Figure 2b to move the sample fluid in a direction from the capillary 7' toward the split junction 30 and the fluid path diverter valve 8, through the fluid path diverter valve 8, and then through the fluid tube 11 to the split junction 12. By switching the fluid path diverter valve 8 in this manner, another relative differential pressure measurement may be taken of a new test sample flowing as per the configuration illustrated in Figure 2b. Reversing the direction of flow through the capillary 7' decreases or eliminates the unwanted breakthrough peak associated with the flow of the sample solution through that capillary, thus reducing the time needed for a successive analysis of another sample.

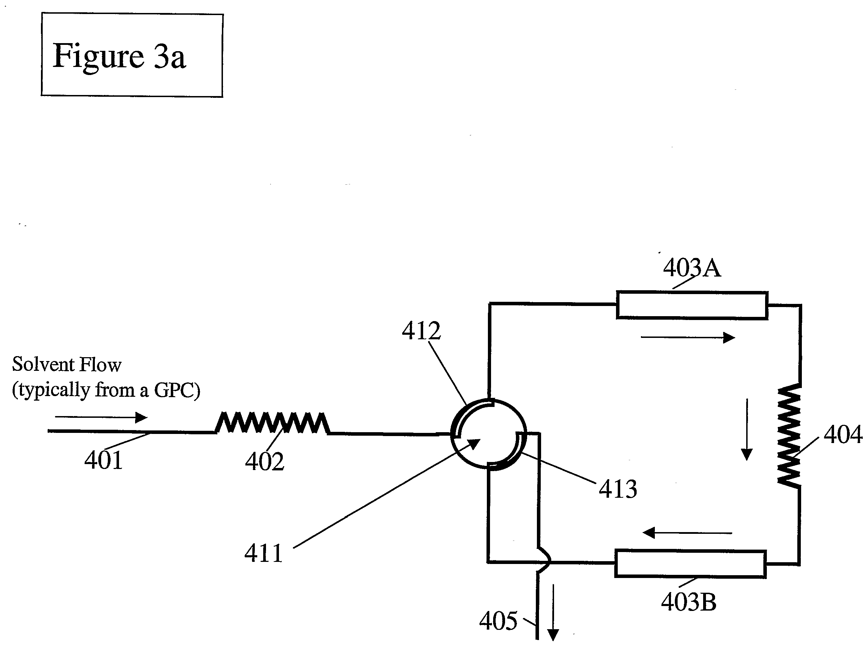

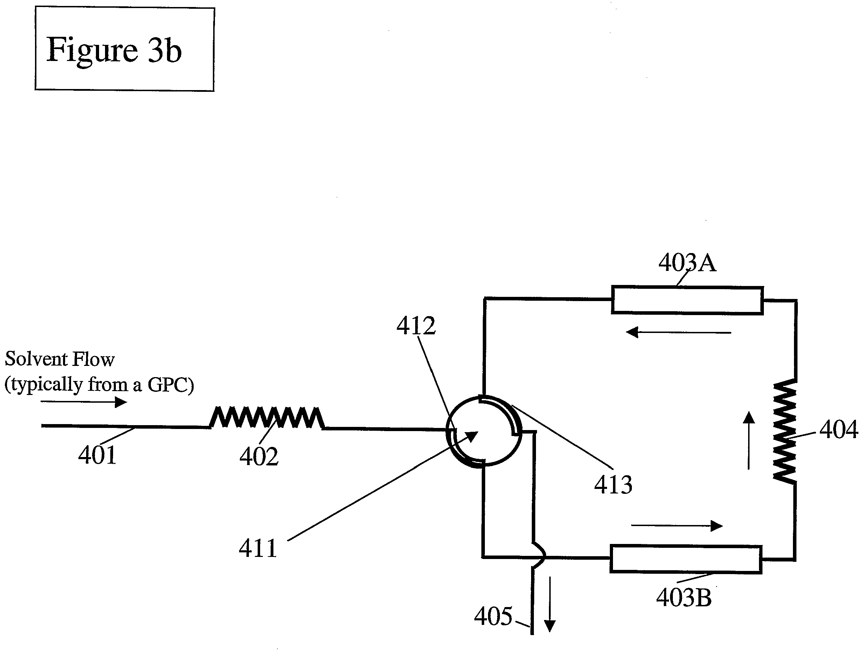

Figures 3a and 3b represent one embodiment of a subcircuit of the apparatus of the present invention in alternative positions. A second delay volume component 403B, is added to the viscometer flowpath after the reference capillary 404. Additionally, a fluid diverting component 411 is included to select which of the two delay volume components 403A and 403B is before and which is after the reference capillary 404. Transducers may be arranged as known in the art for providing the pressure inputs to the transducer diaphragms, whereby the electrical signals provided by the transducers will be proportional to the differential pressure associated with the flow of fluid through the reference capillary and/or the sample capillary for use in the viscosity analysis. The calculation of relative viscosity may then follow that known for the 2-capillary viscometer.

Figure 3a represents the embodiment of the subcircuit in configuration A. The fluid viscosity analysis circuit has an inlet tube 401 connected to a source of solvent flow (not shown) commonly used in the art. The inlet tube 401 connects to a capillary tube 402 known as the sample capillary, the other end of the sample capillary 402 is connected to a fluid diverting component 411 (shown as a 4-port, 2-position valve). The fluid passes through the valve 411 by way of a pathway 412, thence onwards to the delay volume component 403A via a tube. The other end of the delay volume component 403A is connected to a reference capillary 404 via a tube, the other end of which is connected to the second delay volume component 403B via a tube. The other end of the second delay volume component 403B is connected back to the fluid diverting component 411 from which fluid in the delay volume component 403B leaves the circuit through the pathway 413 and exits via the tube 405.

Figure 3b represents the embodiment of the subcircuit in configuration B. The fluid viscosity analysis circuit is substantially identical to that described above for figure 3a except that the fluid diverting component 411 has been changed to be configured in its alternative position. Thus, the pathway from the entry point at the entry tube 401 to the exit tube 405 is: sample capillary 402, fluid diverting component 411, fluid pathway 412, delay volume component 403B, reference capillary 404, delay volume component 403A, fluid diverting component 411, fluid pathway 413, and exit tube 405.

An example of a method of operation of the present invention is provided. For the purposes of clarity and to aid in understanding the method, the starting configuration is assumed to be that shown in Figure 3a. The instrument is in baseline mode when the fluid pathway has only solvent passing through it. Sample solution is injected or enters the circuit by way of the inlet tube 401. When the sample solution passes through the sample capillary 402, there will be a change in fluid pressure sensed by an appropriately placed transducer (not shown). The simultaneous sensing of pressure across the reference capillary 404, sensed by another appropriately placed transducer (See, for example, Figures 2a and 2b), will still be that due to the passage of the solvent which is flowing from the delay volume component 403A. Thus the relative viscosity may be computed from the transducer signals as described in, for example, the Haney, Abbott, *et al.* or de Coral patents. For example, when used in combination with a refractometer or similar concentration detector as the detection device of a gel permeation (or size exclusion) chromatograph, the transducer signals may also be used to determine other information about the sample's properties such as the molecular weight distribution or molecular size distribution.

The method of operation of the subcircuit illustrated in Figure 3b is identical to the method for Figure 3a, with the following changes. The fluid diverting component 411 is configured to accept fluid from the sample capillary 402 in the pathway 412 as shown in figure 3b. The effect of this configuration of the fluid diverting component 411 is to reverse the position in the fluid pathway of the delay volume components 403A, 403B relative to the reference capillary 404. Thus, the pathway of the fluid from the entry point at the inlet tube 401 becomes: the sample capillary 402, the fluid diverting component 411, the fluid pathway 412, the delay volume component 403B, the reference capillary 404, the delay volume component 403A, the fluid diverting component 411, the fluid pathway 413, and the exit tube 405. Thus the delay volume component 403B is in front of the reference capillary 404 and will provide the local supply of solvent to it. The delay volume component 403A is now downstream of the reference capillary 404. Furthermore, the direction of flow of solvent through the delay volume component 403A is reversed and the sample solution that was passing through it is now flushed out by way of the fluid diverting component 411 via the fluid pathway 413 to the exit pipe 405 where it will take no further part in the analysis process.

Thus, the fluid viscosity analysis circuit may be quickly made ready to accept a further sample for analysis. The fluid pathway may stay in this configuration for the duration of the analysis of the next sample, after which the fluid diverting component 411 may again be activated to align to the alternative configuration. By this time, the delay volume component 403A will be completely replenished with solvent. There is minimal disturbance to the fluid viscosity analysis circuit other than the activation of the fluid diverting component 411 and the consequent reversal of flow direction through a part of the circuit. Depending on the transducer connections, the reversal of flow through the reference capillary 404 may reverse the polarity of the pressure signal, but that effect can be accounted for in processing the transducer signals for a relative viscosity calculation.

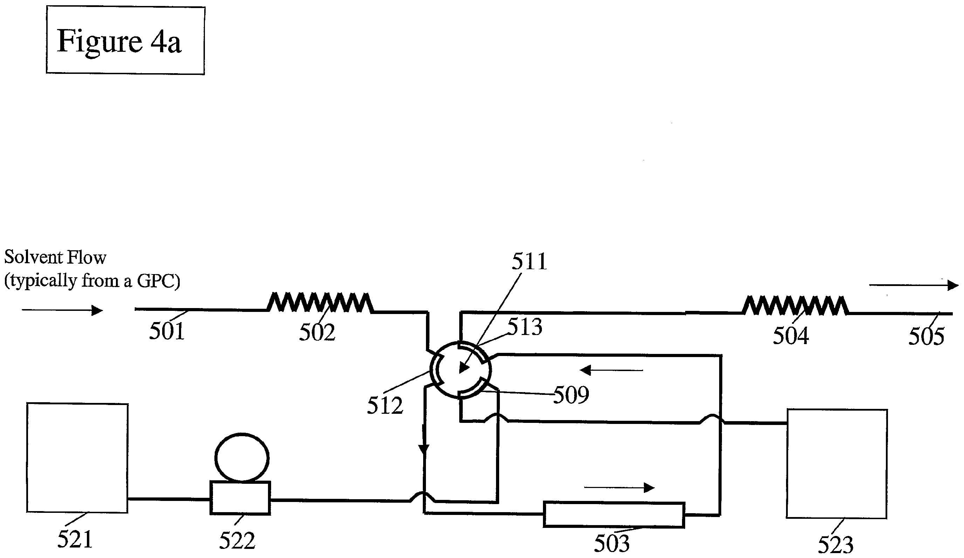

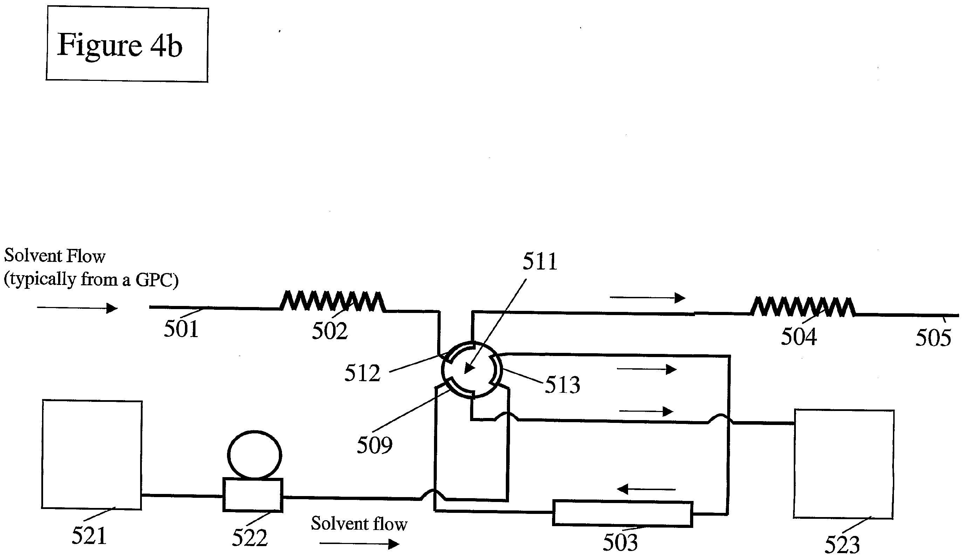

Figures 4a and 4b represent a further embodiment of the apparatus containing a single delay volume component. A further difference from the embodiments in Figures 3a and 3b is that the diverter 511 allows the delay volume component 503 to be connected to either the viscosity measuring circuit or to an external flushing circuit.

Figure 4a represents another embodiment of the apparatus in a first configuration. The fluid viscosity analysis circuit has an inlet tube 501 connected to a source of solvent flow as commonly used in the industry. The inlet tube 501 connects to a capillary tube 502 known as the sample capillary, the other end of which connects to one port of a fluid diverting component 511 (shown as a 6-port, 2-position valve). The fluid pathway passes through the valve by way of a first channel 512, thence onwards to the delay volume component 503. The other end of the delay volume component 503 is connected back to another port of the fluid diverting component 511 and through a second channel 513 to a capillary tube 504 known as the reference capillary, the other end of which leaves the circuit by way of exit tube 505.

An external fluid circuit consists of a solvent source, the purge solvent reservoir 521, connected to a solvent pump 522, connected to the fluid diverting component 511, connected by way of a third channel 509 to the solvent waste vessel 523. The solvent reservoir 521 and the solvent waste vessel 523 may be the same as those used for the main viscometer circuit or can be separate, conventional units. The solvent pump 522 is typically not activated in this mode other than at start-up to dispel any air bubbles that may be present.

The embodiment of the apparatus as disclosed in Figure 4a operates in, but is not limited to, substantially the following manner. The sample solution to be analyzed is injected, in the manner commonly known in the art, into the flowing stream of solvent passing through the viscometer circuit by way of the inlet tube 501 through injection.

When the sample solution passes through the sample capillary 502, there will be a change in fluid pressure sensed by an appropriately placed transducer. The simultaneous analysis of pressure across the reference capillary 504 and the sample capillary 502, analyzed by appropriately placed transducers, will still be that due to the passage of solvent which is flowing from the delay volume component 503. The simultaneous sensing of pressure for each capillary allows the determination of relative viscosity as described in, for example, the Haney, Abbott, *et al.* or de Coral patents. The transducer signals may also be used in combination with the signals produced by a refractometer or similar concentration detector as the detection system of a GPC or SEC to determine other information about the sample's properties such as molecular weight distribution.

Figure 4b represents yet another embodiment of the apparatus in yet another configuration. After the pressure readings are obtained in the analysis configuration , the fluid diverting component 511 may be switched to realign the valve, in a conventional fashion, such that the delay volume component 503 becomes part of the external circuit, while solvent continues to flow in the main viscosity analysis circuit. In this embodiment, the solvent pump 522 is activated to pump solvent through the external circuit by way of the third channel 513 of the fluid diverting component 511 and into the delay volume component 503 leaving by way of the channel 509 of the fluid diverting component 511 to the waste vessel 523 or alternative exit to waste. This way, the sample solution that was passing through the delay volume component 503 is quickly flushed out to waste before it can pass through the reference capillary 504 to cause a breakthrough peak. When the delay volume component 503 is again full of solvent, the solvent pump is switched off and the fluid diverting component 511 may be switched to realign the valve. In this way, the regenerated delay volume component 503 is restored to the analysis.

It may be seen from the preceding description that a new and improved system and method for analysis of fluid properties has been provided. Although very specific examples have been described and disclosed, the embodiments of one form of the apparatus of the instant application is considered to comprise and is intended to comprise any equivalent structure and may be constructed in many different ways to function and operate in the general manner as explained hereinbefore. Accordingly, it is noted that the embodiment of the new and improved system and method described herein in detail for exemplary purposes is of course subject to many different variations in structure, design, application, form, embodiment and methodology. Because many varying and different embodiments may be made within the scope of the inventive concepts herein taught, and because many modifications may be made in the embodiments herein detailed in accordance with the descriptive requirements of the law, it is to be understood that the details herein are to be interpreted as illustrative and not in a limiting sense.

## Claims

1. A pressure sensing apparatus (23) for obtaining signals for analyzing fluid samples, comprising a flow circuit, said flow circuit comprising the following components:
(a) a plurality of capillaries (4, 7, 4', 7'; 402, 404; 502, 504) including at least one sample capillary (4, 4',7; 402; 502) for receiving a sample fluid; said plurality of capillaries (4, 7, 4', 7'; 402, 404; 502, 504) further including a reference capillary (7'; 404; 504) for receiving a reference fluid;
(b) at least one delay volume component (9, 9'; 403A, 403B; 503) connected in the same flow path as the reference capillary (7'; 404; 504);
(c) an inlet (1,401,501);
(d) a first pressure transducer (10) operable to sense a differential pressure associated with the flow of reference fluid through the reference capillary (7'; 404; 504);
(e) a second pressure transducer (6) operable to sense a differential pressure associated with the flow of sample fluid through the at least one sample capillary (4,4',7;402;502) and
(f) an outlet (32,405,523); **characterised in that** the flow circuit further comprises
(g) a valve (8;411;511) operable to connect the at least one delay volume component (9,9'; 403A, 403B; 503) with the circuit outlet (32,405,523) and to selectively reverse a direction of fluid flow through the at least one delay volume component (9,9'; 403A, 403B; 503).

2. The apparatus (23) of claim 1, wherein the first or second pressure transducer (10, 6) is connected to permit the reference or sample fluid to flow through said transducer (10, 6).

3. The apparatus (23) of claim 1, wherein the first pressure transducer (10) is directly connected to opposite ends of the reference capillary (7', 404; 504) for sensing the differential pressure associated with the flow of fluid through said reference capillary (7', 404; 504).

4. The apparatus (23) of claim 1, wherein the second pressure transducer (6) is connected to sense a differential pressure associated with the flow of fluid through the at least one sample capillary (4, 4') in combination with a differential pressure associated with the flow of fluid through the at least one delay volume component (9, 9').

5. The apparatus (23) of claim 1 wherein said valve (8; 411) is operable to alter the direction of flow through said at least one reference capillary (7'; 404).

6. The apparatus (23) of claim 1, wherein at least one of the plurality of capillaries (4, 7, 4', 7'; 402, 404; 502, 504) is connected to a fluid flow path (1; 401; 501) that includes a GPC or SEC column set.

7. The apparatus of claim 1 further comprising: at least one of a reservoir (521) and a pump (522) for delivering reference fluid to said at least one delay volume component (503).

8. The apparatus of claim 1, wherein the first transducer (10) is operable to sense a differential pressure associated with the flow of fluid through the reference capillary (7') arranged in series or parallel with a sample capillary (4', 7).

9. A method for sensing pressure for obtaining signals for analyzing fluid samples, providing a flow circuit, said flow circuit comprising the following components:
(a) a plurality of capillaries (4, 7, 4', 7'; 402, 404; 502, 504) including at least one sample capillary (4, 4',7; 402; 502) for containing a sample fluid,said plurality of capillaries (4, 7, 4', 7'; 402, 404; 502, 504) including a reference capillary (7'; 404; 504) for containing a reference fluid;
(b) at least one delay volume component (9, 9'; 403A, 403B; 503) connected in the same flow path as the at least one reference capillary (7'; 404; 504);
(c) a valve (8; 411; 511) operable to connect the at least one delay volume component (9,9'; 403A, 403B; 503) with the circuit outlet (32,405,523) and to selectively reverse a direction of fluid flow through the at least one delay volume component (9, 9'; 403A, 403B; 503);
(d) a first pressure transducer (10) operable to sense a differential pressure associated with the flow of reference fluid through the reference capillary (7'; 404; 504);
(e) a second pressure transducer (6) operable to sense a differential pressure associated with the flow of sample fluid through the at least one sample capillary (4,4',7;402;502);
(f) an inlet (1,401,501) and
(g) an outlet (32,405,523), the method further comprising the following steps:
(i) introducing from the inlet (1,401,501) a flowing reference fluid to the reference capillary (7'; 404; 504);
(ii) introducing from the inlet (1,401,501) a flowing sample solution to at least one sample capillary (4, 4',7; 402; 502);
(iii) measuring a change in pressure by the first and second pressure transducers as the sample solution flows through the at least one sample capillary (4, 4',7; 402; 502);
(iv) operating the valve (8; 411; 511) to reverse the direction of fluid flow through the at least one delay volume component (9, 9'; 403A, 403B; 503) and thereby purge the sample from the delay volume component (9, 9'; 403A, 403B; 503).

## Patentansprüche

1. Druckmessvorrichtung (23) zum Erhalten von Signalen zum Analysieren von Fluidproben, umfassend einen Strömungskreislauf, wobei der Strömungskreislauf die folgenden Komponenten umfasst:
(a) eine Vielzahl von Kapillaren (4, 7, 4', 7'; 402, 404; 502, 504), die mindestens eine Probenkapillare (4, 4', 7; 402; 502) zur Aufnahme eines Probenfluids umfassen; wobei die Vielzahl von Kapillaren (4, 7, 4', 7'; 402, 404; 502, 504) ferner eine Referenzkapillare (7'; 404; 504) zur Aufnahme eines Referenzfluids umfasst;
(b) mindestens eine Verzögerungsvolumenkomponente (9, 9'; 403A, 403B; 503), die mit dem gleichen Strömungsweg wie die Referenzkapillare (7'; 404; 504) verbunden ist;
(c) einen Einlass (1, 401, 501);
(d) einen ersten Druckwandler (10), der betreibbar ist, um einen Differenzdruck zu erfassen, der mit dem Fluss des Referenzfluids durch die Referenzkapillare (7'; 404; 504) einhergeht;
(e) einen zweiten Druckwandler (6), der betreibbar ist, um einen Differenzdruck zu erfassen, der mit dem Fluss des Probenfluids durch die mindestens eine Probenkapillare (4, 4'; 7; 402; 502) einhergeht; und
(f) einen Auslass (32, 405, 523);
**dadurch gekennzeichnet, dass** der Strömungskreislauf ferner umfasst
(g) ein Ventil (8; 411; 511), das betreibbar ist, um die mindestens eine Verzögerungsvolumenkomponente (9, 9'; 403A, 403B; 503) mit dem Kreislaufauslass (32, 405, 523) zu verbinden und selektiv eine Richtung des Fluidstroms durch die mindestens eine Verzögerungsvolumenkomponente (9, 9'; 403A, 403B; 503) umzukehren.

2. Vorrichtung (23) nach Anspruch 1, wobei der erste oder zweite Druckwandler (10, 6) angeschlossen sind, um zu ermöglichen, dass das Referenz- oder Probenfluid durch den Wandler (10, 6) strömt.

3. Vorrichtung (23) nach Anspruch 1, wobei der erste Druckwandler (10) direkt mit gegenüberliegenden Enden der Referenzkapillare (7', 404; 504) verbunden ist, um den mit dem Fluidstrom durch die Referenzkapillare (7', 404; 504) einhergehenden Differenzdruck zu erfassen.

4. Vorrichtung (23) nach Anspruch 1, wobei der zweite Druckwandler (6) verbunden ist, um einen mit dem Fluidstrom durch die mindestens eine Probenkapillare (4, 4') einhergehenden Differenzdruck in Kombination mit einem mit dem Fluidstrom durch die mindestens eine Verzögerungsvolumenkomponente (9, 9') einhergehenden Differenzdruck zu erfassen.

5. Vorrichtung (23) nach Anspruch 1, wobei das Ventil (8; 411) betreibbar ist, um die Strömungsrichtung durch die mindestens eine Referenzkapillare (7'; 404) zu ändern.

6. Vorrichtung (23) nach Anspruch 1, wobei mindestens eine der Vielzahl von Kapillaren (4, 7, 4', 7'; 402, 404; 502, 504) mit einem Fluidströmungsweg (1; 401; 501) verbunden ist, der einen GPC- oder SEC-Säulensatz umfasst.

7. Vorrichtung nach Anspruch 1, ferner umfassend: mindestens eines aus einem Behälter (521) und einer Pumpe (522) zum Zuführen von Referenzfluid zu der mindestens einen Verzögerungsvolumenkomponente (503).

8. Vorrichtung nach Anspruch 1, wobei der erste Wandler (10) betreibbar ist, um einen mit dem Fluidstrom durch die Referenzkapillare (7') einhergehenden Differenzdruck zu erfassen, die in Reihe oder parallel zu einer Probenkapillare (4', 7) angeordnet ist.

9. Verfahren zum Erfassen von Druck zum Erhalten von Signalen zum Analysieren von Fluidproben, das einen Strömungskreislauf vorsieht, wobei der Strömungskreislauf die folgenden Komponenten umfasst:
(a) eine Vielzahl von Kapillaren (4, 7, 4', 7'; 402, 404; 502, 504), die mindestens eine Probenkapillare (4, 4', 7; 402; 502) zur Aufnahme eines Probenfluids umfasst; wobei die Vielzahl von Kapillaren (4, 7, 4', 7'; 402, 404; 502, 504) ferner eine Referenzkapillare (7'; 404; 504) zur Aufnahme eines Referenzfluids umfasst;
(b) mindestens eine Verzögerungsvolumenkomponente (9, 9'; 403A, 403B; 503), die mit dem gleichen Strömungsweg wie die mindestens eine Referenzkapillare (7'; 404; 504) verbunden ist;
(c) ein Ventil (8; 411; 511), das betreibbar ist, um die mindestens eine Verzögerungsvolumenkomponente (9, 9'; 403A, 403B; 503) mit dem Kreislaufauslass (32, 405, 523) zu verbinden und selektiv eine Richtung des Fluidstroms durch die mindestens eine Verzögerungsvolumenkomponente (9, 9'; 403A, 403B; 503) umzukehren;
(d) einen ersten Druckwandler (10), der betreibbar ist, um einen Differenzdruck zu erfassen, der mit dem Fluss des Referenzfluids durch die Referenzkapillare (7'; 404; 504) einhergeht;
(e) einen zweiten Druckwandler (6), der betreibbar ist, um einen Differenzdruck zu erfassen, der mit dem Fluss des Probenfluids durch die mindestens eine Probenkapillare (4, 4'; 7; 402; 502) einhergeht;
(f) einen Einlass (1, 401, 501); und
(g) einen Auslass (32, 405, 523), wobei das Verfahren ferner die folgenden Schritte umfasst:
(i) Einbringen eines strömenden Referenzfluids von dem Einlass (1, 401, 501) in die Referenzkapillare (7'; 404; 504);
(ii) Einbringen einer strömenden Probenlösung von dem Einlass (1, 401, 501) in mindestens eine Probenkapillare (4, 4', 7; 402; 502);
(iii) Messen einer Druckänderung durch den ersten und zweiten Druckwandler, wenn die Probenlösung durch die mindestens eine Probenkapillare (4, 4', 7; 402; 502) strömt;
(iv) Betätigen des Ventils (8; 411; 511), um die Richtung des Fluidstroms durch die mindestens eine Verzögerungsvolumenkomponente (9, 9'; 403A, 403B; 503) umzukehren und dadurch die Probe aus der Verzögerungsvolumenkomponente (9, 9'; 403A, 403B; 503) zu spülen.

## Revendications

1. Appareil de détection de pression (23) pour obtenir des signaux pour analyser des échantillons de fluide, comprenant un circuit d'écoulement, ledit circuit d'écoulement comprenant les composants suivants:
(a) une pluralité de capillaires (4, 7, 4', 7'; 402, 404; 502, 504) comprenant au moins un capillaire d'échantillon (4, 4', 7; 402; 502) pour recevoir un échantillon de fluide; la pluralité de capillaires (4, 7, 4', 7'; 402, 404; 502, 504) comprenant en outre au moins un capillaire de référence (7'; 404; 504) pour recevoir un fluide de référence;
(b) au moins un composant de volume de retard (9, 9'; 403A, 403B; 503) connecté dans le même chemin d'écoulement que le capillaire de référence (7'; 404, 504);
(c) une entrée (1, 401, 501);
(d) un premier transducteur de pression (10) actionnable pour détecter une pression différentielle associée à l'écoulement de fluide de référence à travers le capillaire de référence (7'; 404; 504);
(e) un second transducteur de pression (6) actionnable pour détecter une pression différentielle associée à l'écoulement de fluide d'échantillon à travers ledit au moins un capillaire d'échantillon (4, 4', 7; 402; 502); et
(f) une sortie (32, 405, 523),
**caractérisé en ce que** le circuit d'écoulement comprend en outre:
(g) une soupape (8; 411; 511) actionnable pour connecter ledit au moins un composant de volume de retard (9, 9'; 403A, 403B; 503) à la sortie de circuit (32, 405, 523) et pour inverser de façon sélective une direction d'écoulement de fluide à travers ledit au moins un composant de volume de retard (9, 9'; 403A, 403B; 503).

2. Appareil (23) selon la revendication 1, dans lequel le premier ou le second transducteur de pression (10, 6) est connecté de manière à permettre à un fluide de référence de s'écouler à travers ledit transducteur (10, 6).

3. Appareil (23) selon la revendication 1, dans lequel le premier transducteur de pression (10) est connecté directement à des extrémités opposées du capillaire de référence (7', 404; 504) dans le but de détecter une pression différentielle associée à l'écoulement de fluide à travers ledit capillaire de référence (7', 404; 504).

4. Appareil (23) selon la revendication 1, dans lequel le second transducteur de pression (6) est connecté dans le but de détecter une pression différentielle associée à l'écoulement de fluide à travers au moins un capillaire d'échantillon (4, 4') en combinaison avec une pression différentielle associée à l'écoulement de fluide à travers un composant de volume de retard (9, 9').

5. Appareil (23) selon la revendication 1, dans lequel ladite soupape (8; 411) est actionnable pour modifier la direction d'écoulement à travers ledit au moins un capillaire de référence (7'; 404).

6. Appareil (23) selon la revendication 1, dans lequel au moins un de la pluralité de capillaires (4, 7, 4', 7'; 402; 502, 504) est connecté à un chemin d'écoulement de fluide (1; 401; 501) qui comprend un ensemble de colonnes GPC ou SEC.

7. Appareil selon la revendication 1 comprenant en outre au moins un élément parmi un réservoir (521) et une pompe (522) pour distribuer un fluide de référence audit au moins un composant de volume de retard (503).

8. Appareil selon la revendication 1, dans lequel le premier transducteur (10) est actionnable pour détecter une pression différentielle associée à l'écoulement de fluide à travers le capillaire de référence (7') agencé en série ou en parallèle avec un un capillaire d'échantillon (4', 7).

9. Procédé pour détecter une pression dans le but d'obtenir des signaux pour analyser des échantillons de fluide, fournissant un circuit d'écoulement, ledit circuit d'écoulement comprenant les composants suivants:
(a) une pluralité de capillaires (4, 7, 4', 7'; 402, 404; 502, 504) comprenant au moins un capillaire d'échantillon (4, 4', 7; 402; 502) pour contenir un échantillon de fluide; la pluralité de capillaires (4, 7, 4', 7'; 402, 404; 502, 504) comprenant un capillaire de référence (7'; 404; 504) pour contenir un fluide de référence;
(b) au moins un composant de volume de retard (9, 9'; 403A, 403B; 503) connecté dans le même chemin d'écoulement que le capillaire de référence (7'; 404, 504);
(c) une soupape (8; 411; 511) actionnable pour connecter ledit au moins un composant de volume de retard (9, 9'; 403A, 403B; 503) à la sortie de circuit (32, 405, 523) et pour inverser de façon sélective une direction d'écoulement de fluide à travers ledit au moins un composant de volume de retard (9, 9'; 403A, 403B; 503);
(d) un premier transducteur de pression (10) actionnable pour détecter une pression différentielle associée à l'écoulement de fluide de référence à travers le capillaire de référence (7'; 404; 504);
(e) un second transducteur de pression (6) actionnable pour détecter une pression différentielle associée à l'écoulement de fluide d'échantillon à travers ledit au moins un capillaire d'échantillon (4, 4', 7; 402; 502);
(f) une entrée (1, 401, 501); et
(g) une sortie (32, 405, 523),
le procédé comprenant en outre les étapes suivantes:
(i) introduire à partir de l'entée (1, 401, 501) un fluide de référence s'écoulant vers le capillaire de référence (7'; 404; 504)
(ii) introduire à partir de l'entrée (1, 401, 501) une solution d'échantillon s'écoulant vers ledit au moins un capillaire d'échantillon (4, 4', 7; 402; 502) ;
(iii) mesurer une variation de pression à l'aide des premier et second transducteurs de pression lorsque la solution d'échantillon s'écoule à travers ledit au moins un capillaire d'échantillon (4, 4', 7; 402; 502); et
(iv) actionner la soupape (8; 411; 511) afin d'inverser la direction d'écoulement de fluide à travers ledit au moins un composant de volume de retard (9, 9'; 403A, 403B; 503) et de purger ainsi l'échantillon hors du composant de volume de retard (9, 9'; 403A, 403B; 503).
